Europäisches Patentamt

European Patent Office

Office européen des brevets

(11) Veröffentlichungsnummer: **0 303 773**
**A2**

(12) **EUROPÄISCHE PATENTANMELDUNG**

(21) Anmeldenummer: 88107772.1

(22) Anmeldetag: 14.05.88

(51) Int. Cl.⁴: **A61B 17/58**

(30) Priorität: 20.08.87 DE 8711317 U

(43) Veröffentlichungstag der Anmeldung:
22.02.89 Patentblatt 89/08

(84) Benannte Vertragsstaaten:
AT CH DE ES FR GB IT LI NL

(71) Anmelder: **Howmedica GmbH**
**Professor-Küntscher-Str. 1-5**
**D-2314 Schönkirchen ü. Kiel(DE)**

(72) Erfinder: **Blauth, Michael, Dr. med.**
**Einsteinstr. 37**
**D-3000 Hannover 51(DE)**
Erfinder: **Harder, Hans, E.**
**Mecklenburger Strasse 37**
**D-2316 Probsteierhagen(DE)**

(74) Vertreter: **Dipl.-Ing. H. Hauck Dipl.-Phys. W.**
**Schmitz Dipl.-Ing. E. Graalfs Dipl.-Ing. W.**
**Wehnert Dr.-Ing. W. Döring**
**Neuer Wall 41**
**D-2000 Hamburg 36(DE)**

(54) Stützvorrichtung für die Wirbel der menschlichen Wirbelsäule.

(57) Stützvorrichtung für die Wirbel der menschlichen Wirbelsäule, mit Haltekörpern, von denen Schanz'sche Schrauben klemmend aufgenommen werden, einem Verbindungselement für die Haltekörper, auf denen die Haltekörper in ihrer Relativlage verstellbar gelagert, jedoch in einer vorgegebenen Relativlage feststellbar sind, wobei die Haltekörper eine Gewindehülse aufweisen, die auf einem Ende eines Gewindebolzens aufschraubbar ist, der Gewindebolzen an den Enden gegenläufige Gewinde aufweist, im Haltekörper eine Haltebuchse drehbar, jedoch axial unverstellbar gelagert ist, die gegebenenfalls Kraftangriffsflächen für ein Werkzeug aufweist, die Haltebuchse eine Schanz'sche Schraube aufnehmende Querdurchbohrung aufweist, die mit einer Bohrung im Haltekörper ausrichtbar ist, und im Haltekörper eine mit der Haltebuchse zusammenwirkende Feststellschraube angeordnet ist, die die Haltebuchse in einer eingenommenen Drehlage und zugleich die Schanz'sche Schraube klemmend festlegt.

FIG.1

## Stützvorrichtung für die Wirbel der menschlichen Wirbelsäule

Die Neuerung bezieht sich auf eine Stützvorrichtung für die Wirbel der menschlichen Wirbelsäule nach dem Oberbegriff des Anspruchs 1.

Beim Bruch eines Wirbels wird dieser unterschiedlich stark verformt. Dabei kann es zu Verkrümmungen der Wirbelsäule und zu einer druckbedingten Verletzung des Rückenmarkes kommen. Für eine möglichst weitgehende Wiederherstellung des Patienten sollte der Bruch reponiert wird. Dafür müssen die angrenzenden Wirbel verlagert werden, um die Fraktursegmente in die lagerichtige Position zurückzubringen. Ohne zusätzliche Stützvorrichtungen ist eine langdauernde unbewegliche Lagerung des Patienten erforderlich. Um die Repositionsmöglichkeiten zu verbessern und die langdauernde Ruhigstellung der Wirbelsäule zu vermeiden, sind bereits Stützvorrichtungen interner und externer Art bekannt geworden. Die bekannten Stützvorrichtungen verwenden sogenannte Schanz'sche Schrauben. Die Schanz'schen Schrauben haben eine beträchtliche Länge und sind an einem Ende mit einem selbstschneidenden Gewinde versehen. Die Schanz'schen Schrauben werden von dorsal über den Bogenfuß in den Wirbelkörper eingeschraubt. Bei der Reposition dienen die eingeschraubten Schanz'schen Schrauben als Hebel, mit denen die dem gebrochenen Wirbel benachbarten Wirbel zumeist auseinanderbewegt werden, um den zerbrochenen Wirbel zu reponieren und im Anschluß zu entlasten. Nach der Reposition werden die Schanz'schen Schrauben in einer entsprechenden Haltevorrichtung in ihrer Winkellage und in ihrem Abstand zueinander festgestellt. Die über die Vorrichtung hinausragenden Enden der Schanz'schen Schrauben werden teils mit Hilfe geeigneter Zangen abgekniffen und teils durch Lösen vorgesehener Verbindungsstellen entfernt.

Es ist bereits eine Stützvorrichtung bekannt geworden, bei der die Haltevorrichtung aus zwei parallelen Gewindestangen besteht, auf denen Haltekörper angeordnet sind. Die Haltekörper können durch Drehung von Gewindemuttern in ihrer Relativlage zueinander verändert werden. Ist eine gewünschte Position erreicht, wird die Spindelmutter durch eine Kontermutter festgesetzt. Die Haltekörper nehmen in den entsprechenden Bohrungen die Schanz'schen Schrauben auf, wobei jeder Haltekörper zur Aufnahme von zwei Schanz'schen Schrauben ausgebildet ist.

Die bekannte Stützvorrichtung baut besonders im Bereich der Enden der Haltekörper verhältnismäßig groß, was für die interne Anordnung nachteilig und für den Patienten unbequem ist. Außerdem müssen während des Einbauvorgangs zahlreiche Schrauben ein- und festgedreht werden. Dies kann den Eingriff komplizieren und verlängern.

Die Schanz'schen Schrauben werden, wie bereits erwähnt, nach der Reposition gekürzt. Naturgemäß bleibt hierbei ein über die Haltevorrichtung überstehendes Ende stehen, das zudem mit einem mehr oder weniger scharfen Grat versehen ist. Das überstehende Ende vergrößert die gesamte Baugröße der Stützvorrichtung. Der scharfe Grat kann das Gewebe reizen und zu Unverträglichkeiten führen.

Der Neuerung liegt daher die Aufgabe zugrunde, eine Stützvorrichtung für die Wirbel der menschlichen Wirbelsäule zu schaffen, die möglichst klein baut, um so gering wie möglich aufzutragen. Die Stützvorrichtung soll einfach zu bedienen und die Schanz'schen Schrauben zweigeteilt sein. Der Überstand der Schanz'schen Schrauben soll möglichst gering ausfallen.

Diese Aufgabe wird durch die Merkmale des Kennzeichnungsteils des Anspruches 1 gelöst.

Bei der neuerungsgemäßen Stützvorrichtung werden zur Abstützung von 3 Wirbeln zwei getrennte Stützvorrichtungen vorgesehen, die beidseits des Dornfortsatzes sehr nahe am Wirbelbogen plaziert werden können. Jede Vorrichtung weist zwei Haltekörper auf, die jeweils eine Gewindehülse aufweisen. Die Gewindehülsen sitzen auf entgegengesetzten Enden des Gewindebolzens, der gegenläufige Gewinde aufweist. Haltekörper und Gewindebolzen bilden daher ein an sich bekanntes Spannschloß.

Jeder Haltekörper ist mit einer Haltebuchse versehen, die drehbar im Haltekörper gelagert ist und Kraftangriffsflächen für ein Werkzeug aufweist, beispielsweise einen Sechskant. Die Haltebuchse ist mit einer eine Schanz'sche Schraube aufnehmenden Querdurchbohrung versehen. Die Querdurchbohrung ist mit einer Bohrung am Haltekörper ausrichtbar, und der Haltebuchse ist eine Feststellschraube zugeordnet, die die Haltebuchse in einer eingenommenen Drehlage und zugleich die Schanz'sche Schraube klemmend festlegt. Die Haltekörper werden auf die in den entsprechenden Wirbel eingeschraubten Schanz'schen Schrauben aufgesteckt. Dabei muß zuvor der notwendige Längsabstand der Haltebuchsen über die Relativverdrehung von Gewindestange und Gewindehülsen eingestellt werden. Die Reposition kann in herkömmlicher Weise durchgeführt werden, indem die überstehenden Enden der Schanz'schen Schrauben erfaßt und vorzugsweise aufeinander zu bewegt werden, um die erfaßten Wirbel voneinander zu entfernen. Vorteilhafter ist es indessen, von vornherein so kurze Schanz'sche Schrauben zu verwenden, daß sie soeben mit ihren Enden von der Halterbuchse aufgenommen werden. Die Kraftaufbringung für die Reposition erfolgt an den Kraftangriffsflächen der Haltebuchse. Ist die Reposition durchgeführt, werden die Feststellschrauben angezogen, so daß die Drehlage der Haltebuchsen fixiert wird. Gleichzeitig hiermit erfolgt die axiale Festlegung der Schanz'schen Schraube in der Haltebuchse. Zu diesem Zweck ist die Haltebuchse vorzugsweise geschlitzt, und zwar vorzugsweise diametral.

Da die Schanz'schen Schrauben beim Repositionsvorgang eine Schwenkbewegung ausführen, ist es nach einer weiteren Ausgestaltung der Neuerung zweckmäßig, wenn sich die Durchbohrung im Haltekörper von der Mitte ausgehend nach außen jeweils in Achsrichtung erweitert.

Bei der Neuerung ist von der Erkenntnis ausgegangen worden, daß es für die Reposition und Versorgung einer gebrochenen Wirbelsäulenpartie ausreicht, zwei separate Stützvorrichtungen vorzusehen. Sie können durch ihren kleinbauenden Aufbau nahe an den Wirbelbogen herangebracht werden und lassen sich im umgebenden Muskelgewebe gut und mit verringerter Gefahr der Reizung unterbringen.

Die Fixierung der Schanz'schen Schraube in der Haltebuchse und der Haltebuchse in ihrer Winkellage wird mit Hilfe einer Feststellschraube erreicht. Hierfür kann eine platzsparende Madenschraube verwendet werden, die versenkt im Kopf des Haltekörpers eingeschraubt wird. Eine andere alternative Ausführungsform sieht einen innerhalb des Haltekörpers die Haltebuchse umgebenden Spannring vor, der einen radial nach außen weisenden Gewindeansatz besitzt. Der Gewindeansatz wirkt mit einer außen liegenden Klemmschraube zusammen. Eine Durchbohrung erstreckt sich durch den Gewinde ansatz, die Klemmschraube und diametral durch den Spannring für die Aufnahme einer Schanz'schen Schraube. Der Haltekörper weist im Bereich der Durchbohrung für die Schanz'sche Schraube eine konvex gewölbte Klemmfläche auf, und zwischen der Klemmfläche und der Klemmschraube ist ein Klemmring angeordnet, dessen der Klemmfläche zugewandte Seite entsprechend konkav gewölbt ist. Wird die Klemmschraube festgezogen, drückt der Klemmring gegen die konvexe Klemmfläche des Haltekörpers und fixiert dadurch die eingenommene Winkellage der Durchbohrung und damit der Schanz'schen Schraube. Gleichzeitig wird der Klemmring relativ zur Haltebuchse verstellt, so daß auch die Haltebuchse in der Bohrung im Klemmkörper festgelegt wird. Die zusammenwirkenden Klemmflächen weisen vorzugsweise eine Zahnung auf, die eine große Kraftaufnahme zulassen. Die zuletzt beschriebene Ausführungsform ist ohnehin zur Aufnahme großer Kräfte geeignet, ohne daß die Fixierung nachgibt.

Außerdem hat sie den Vorteil, daß die Schlitzung der Haltebuchse stets günstig zur Spannrichtung liegt, d.h. stets mit optimaler Kraft beaufschlagt wird, um die Schanz'sche Schraube wirksam in der Buchse festzuklemmen.

Die Erfindung wird nachfolgend anhand von Zeichnungen näher erläutert.

Fig. 1 zeigt die Draufsicht auf einen schematisch dargestellten Lendenwirbel und einen Brustwirbel.

Fig. 2 zeigt die Seitenansicht einer neuerungsgemäßen Stützvorrichtung.

Fig. 3 zeigt die um 90° verdrehte Ansicht der Stützvorrichtung nach Fig. 2 teilweise im Schnitt.

Fig. 4 zeigt eine Seitenansicht eines Haltekörpers der Stützvorrichtung nach Fig. 2 in Richtung Pfeil 4.

Fig. 5 zeigt einen Schnitt durch die Darstellung nach Fig. 4 entlang der Linie 5-5.

Fig. 6 zeigt vergrößert eine Haltebuchse der Stützvorrichtung nach den Fign. 2 oder 3.

Fig. 7 zeigt eine Ansicht der Haltebuchse nach Fig. 6 in Richtung Pfeil 7.

Fig. 8 zeigt einen Schnitt durch eine andere Ausführungsform eines Haltekörpers.

Fig. 9 zeigt die gegenüber Fig. 8 um 90° verdreht Seitenansicht des Haltekörpers teilweise im Schnitt.

Fig. 10 zeigt die Seitenansicht des Haltekörpers nach Fig. 8 in Richtung Pfeil 10, jedoch ohne Spannring und Klemmschraube.

Fig. 11 zeigt ein Teil einer Schanz'schen Schraube mit einem Werkzeug zur Betätigung der Schanz'schen Schraube.

Fig. 12 zeigt eine Seitenansicht der Darstellung nach Fig. 11 in Richtung Pfeil 12.

Bevor auf die in den Zeichnungen dargestellten Einzelheiten näher eingegangen wird, sei vorangestellt, daß jedes der beschriebenen Merkmale für sich oder in Verbindung mit Merkmalen der Ansprüche von neuerungswesentlicher Bedeutung ist.

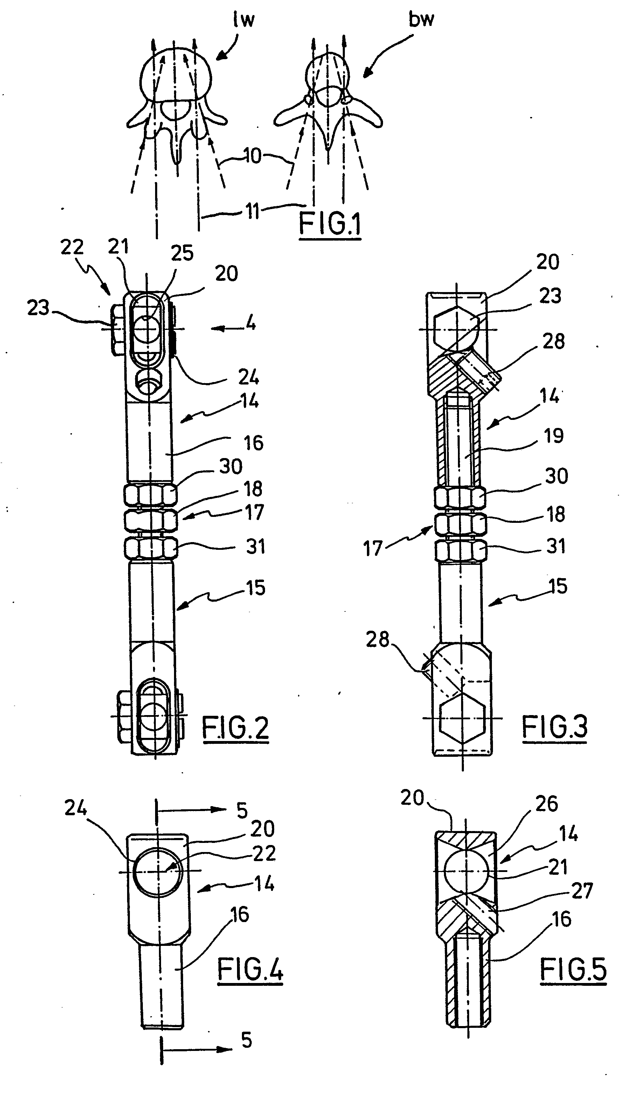

Fig. 1 veranschaulicht einen Lendenwirbel lw bzw. einen Brustwirbel bw schematisch in Draufsicht. Die gestrichelten Linien 10 deuten Achsen für Bohrungen an, die die Schanz'schen Schrauben aufnehmen. Die Bohrungen erstrecken sich zwischen Dornfortsatz und Querfortsatz durch den Wirbelbogenfuß in den Wirbelkörper hinein. Die Bohrungsachsen konvergieren in Richtung Wirbelkörper um etwa 15°. Strichpunktiert sind Achsen 11 dargestellt für Bohrungen ebenfalls durch den Wirbelbogenfuß in den Wirbelkörper hinein. Die Achsen verlaufen annähernd parallel zueinander. Bohrungen in Richtung der Achsen 11 werden von Stützvorrichtungen ermöglicht bzw. benötigt, wie sie nachfolgend beschrieben werden.

Die in den Figuren 2 und 3 dargestellte Stützvorrichtung weist zwei Haltekörper 14, 15 auf. Jeder Haltekörper 14, 15 weist eine Gewindehülse 16 auf (nachfolgend wird nur der Haltekörper 14 beschrieben, da der Haltekörper 15 identisch aufgebaut ist). Die Gewindehülsen sind auf eine Gewindestange 17 geschraubt, die beidseits eines Sechskants 18 zwei Gewindeabschnitte aufweist, von denen einer bei 19 dargestellt ist. Die Gewindeabschnitte 19 haben einen gegenläufiges Gewinde. Entsprechend haben die Gewindehülsen 16 ein dazu passendes Gewinde. Werden die Haltekörper 14, 15 festgehalten und wird die Gewindestange 17 am Sechskant 18 gedreht, bewegen sich die Haltekörper 14, 15 aufeinander zu bzw. voneinander fort. Die Gewindehülsen 16 bilden daher mit der Gewindestange 17 ein an sich bekanntes Spannschloß.

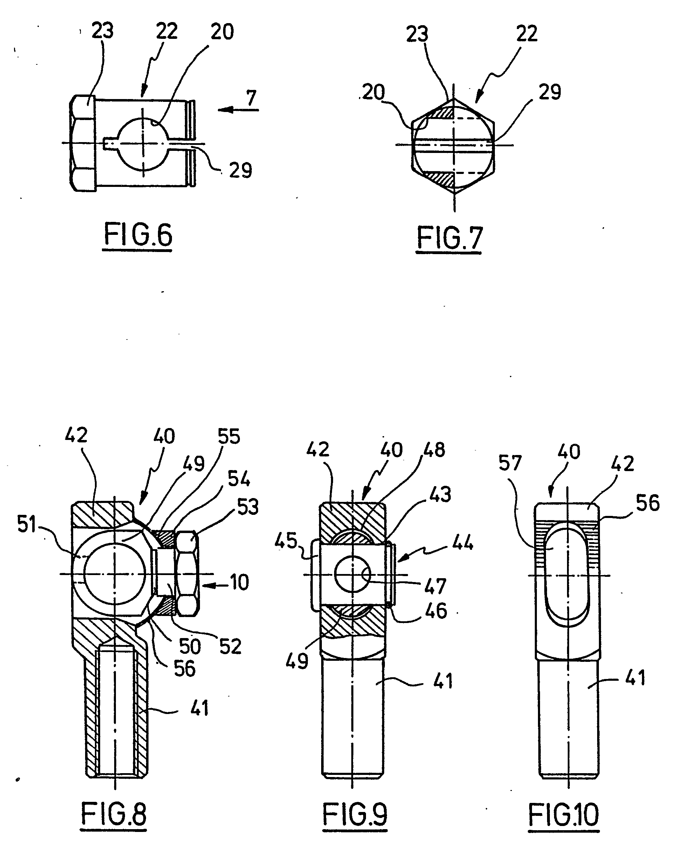

An die Gewindehülsen 16 schließt sich ein Kopf 20 an, der eine quaderförmige Außenkontur hat. Der Kopf 20 weist eine Durchbohrung 21 auf. Die Durchbohrung 21 nimmt eine Haltbuchse 22 auf, die an einem Ende einen Sechskantkopf 23 aufweist, der gegen eine Seitenfläche des Kopfes 20 anliegt. Das andere etwas über die gegenüberliegende Außenfläche des Kopfes 20 überstehende Ende der Buchse 22 wird durch einen Federring 24 gesichert, der gegen die entsprechende Außenfläche anliegt. Die Buchse 22 ist mithin drehbar, jedoch axial gesichert von der Bohrung 21 aufgenommen. Die Haltebuchse 22 weist eine Querdurchbohrung 25 auf. Diese ist ausgerichtet zu einer Bohrung 26 im Kopf 20 die senkrecht verläuft zur Durchbohrung 21. Wie insbesondere aus Fig. 5 zu erkennen, ist die Durchbohrung 26 nach außen in Achsrichtung erweitert, so daß sich außen ein Langloch ergibt, wie in Fig. 2 zu erkennen. Der Kopf 20 weist ferner eine Schrägbohrung 27 auf, die an dem Absatz zwischen der Gewindehülse 16 und dem Kopf 20 beginnt und sich schräg nach außen erstreckt in die Bohrung 26 hinein. Die Bohrung 27 nimmt eine Feststellmadenschraube 28 auf (Fig. 3), die zur Fixierung der Haltebuchse 22 dient.

Die Haltebuchse 22 ist deutlicher aus den Figuren 6 und 7 zu entnehmen. Man erkennt, daß die zylindrische Haltebuchse 22 diametral bei 29 geschlitzt ist, wobei die Schlitzung über die Querbohrung 20 in Richtung Sechskantkopf 23 hinausgeht. Die Querbohrung 20 dient zur Aufnahme der nicht gezeigten Schanz'schen Schraube, die in Richtung der Linien 11 gemäß Fig. 1 in die Wirbel eingeschraubt wird. Wird die Haltebuchse 22 mit Hilfe der Feststellschraube 28 beaufschlagt, wird zum einen die Winkellage der Buchse 22 festgelegt. Ferner wird die Buchse 22 durch die Schlitzung 29 zusammengepreßt, so daß die Schanz'sche Schraube innerhalb der Buchse mit der Bohrung 20 ebenfalls festgeklemmt wird. Der beschriebene Klemmvorgang erfolgt nach der Reponierung der Wirbel. Diese erfolgt ebenfalls mit Hilfe der in den Buchsen 22 aufgenommenen Schanz'schen Schraube, indem ein Werkzeug an dem Sechskantkopf 23 angreift und die Schanz'sche Schraube verschwenkt. Eine Verstellung der Schanz'schen Schrauben relativ zueinander erfolgt auch, wenn die Gewindestange 17 verdreht wird. Ist die gewünschte Position erreicht, können die Haltekörper 14, 15 mit Hilfe von Kontermuttern 30, 31 in bekannter Weise festgestellt werden.

Man erkennt aus den Figuren 2 bis 7, daß zwei der gezeigten Stützvorrichtungen bequem beidseits des Dornfortsatzes des Wirbels nahe am Wirbelbogen angebracht werden können. Die äußeren Abmessungen der gezeigten Stützvorrichtung sind äußerst gering. Die Querausdehnung in der einen Richtung wird durch die Länge der Haltebuchse 22 bestimmt, während die Ausdehnung im rechten Winkel dazu im wesentlichen durch die Mindestbreite bestimmt ist, die für die Aufnahme der Haltebuchse notwendig ist sowie durch den notwendigen Überstand der Feststellschraube 28.

In den Figuren 8 bis 10 sind abgewandelte Haltekörper 40 gezeigt. Sie weisen eine Gewindehülse 41 auf, die der Gewindehülse 16 der vorstehend beschriebenen Ausführungsform gleicht. Auch die Kontur des Kopfes 42 stimmt bis auf eine Abweichung weitgehend mit der der vorstehend beschriebenen Ausführungsform überein. Der Kopf 20 weist eine Durchbohrung 43 auf, die sich quer zur Achse der Gewindehülse 41 erstreckt, wie dies auch bei der oben beschriebenen Ausführungsform der Fall ist. Die Bohrung 43 nimmt eine Haltebuchse 44 auf, die an der einen Seite durch einen Kopf 45 und an der anderen Seite durch einen Federring 46 axial in der Bohrung 43 festgelegt ist. Der Kopf 45 ist jedoch entgegen der obigen Ausführungsform kreisrund gestaltet. Die Haltebuchse 44 weist eine Querdurchbohrung 47 auf zur Aufnahme einer Schanz'schen Schraube. Die Haltebuchse 47 ist ähnlich wie die Buchse nach den Figuren 6 und 7 geschlitzt, was hier jedoch nicht dargestellt ist.

Die Bohrung 43 besitzt im Inneren des Kopfes 42 eine ringnutartige Erweiterung 48, wobei die Kontur der Nut 48 kreisbogenförmig ist. Die Ringnut 48 nimmt einen im Querschnitt angepaßten Spannring 49 auf, der die Haltebuchse 44 umgibt (Fig. 9). Der Spannring 49 weist einen radial nach außen sich erstreckenden Ansatz 50 auf, der mit einem Außengewinde versehen ist und der axial durchbohrt ist. Diese Bohrung ist koaxial mit einer diametral gegenüberliegenden Durchbohrung 51 durch den Spannring 49.

Auf den Ansatz 50 ist eine kurze Gewindehülse 52 einer Klemmschraube 53 geschraubt. Auf dem Hülsenabschnitt 52 sitzt ein Klemmring 54, dessen vom Schraubenkopf wegweisende Seite konkav gewölbt ist, wie bei 55 dargestellt. Aus den Figuren 8 und 10 ist zu erkennen, daß eine Außenseite des Kopfes 42 konvex gewölbt ist, wie bei 56 dargestellt. Die Wölbung weist eine Zahnung auf. Im Bereich der Wölbung erstreckt sich ein Langloch 57. Der Klemmring 49 kann daher verschwenkt werden, da sich der Ansatz 50 bzw. der Hülsenabschnitt 52 durch das Langloch 57 hindurcherstrekken. Die konkav gewölbte Fläche 55 des Klemmrings 54 weist eine entsprechende Zahnung auf.

Die Durchbohrungen im Klemmring 49, im radialen Ansatz 50 und in der Klemmschraube 53 sind zur Bohrung 47 der Haltebuchse 44 ausgerichtet, so daß sich die Schanz'sche Schraube hindurcherstrecken kann. Soll die Schanz'sche Schraube in der Haltebuchse 44 festgelegt und soll die Haltebuchse 44 in ihrer Winkellage fixiert werden, wird die Schraube 53 angezogen. Der Klemmring 54 kommt mit der Klemmfläche 56 in Eingriff, so daß die Winkellage festgelegt ist. Der Klemmring 49 hat sich bei diesem Vorgang relativ zur Längsachse des Haltekörpers 40 verschoben. Dadurch wird ein Querdruck auf die Haltebuchse 44 erzeugt. Da die Buchse 44 geschlitzt ist, wird ihr Innendurchmesser verringert, wodurch die Schanz'sche Schraube festgeklemmt wird. Da die Relativdrehlage von Haltebuchse 44 und Klemmring 49 gleich bleibt, kann die Schlitzung der Buchse 44 so liegen, daß die Kraft optimal auf die Buchse 44 ausgeübt wird, um die Schanz'sche Schraube wirksam festzuklemmen.

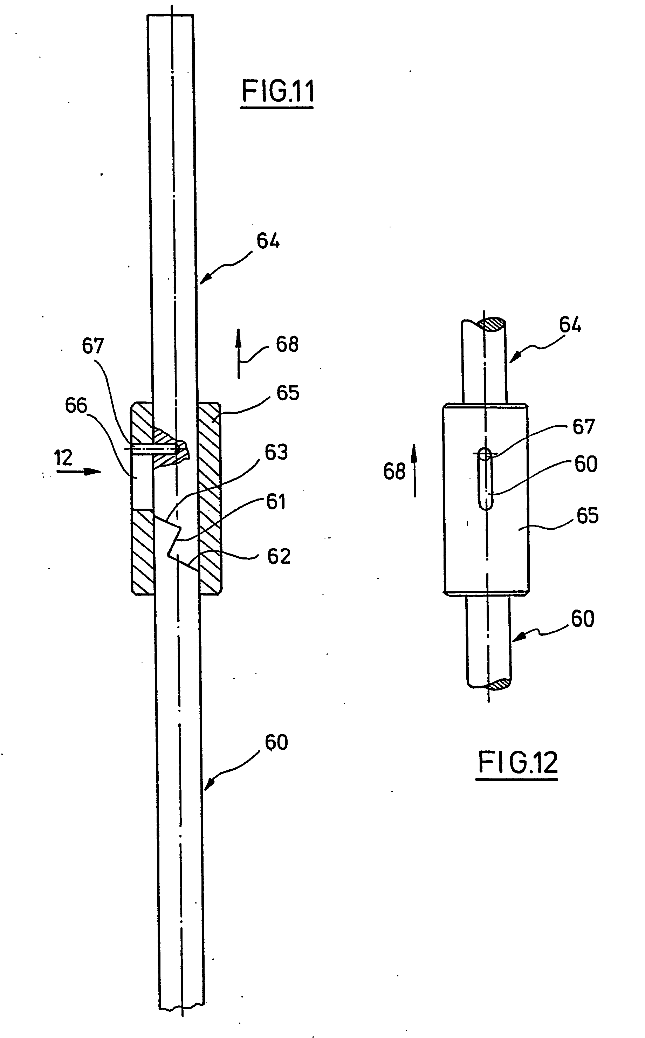

Figuren 11 und 12 zeigen das hintere Ende einer Schanz'schen Schraube 60. Das vordere Ende ist mit einem selbstschneidenden Gewinde versehen. Am hinteren Ende der Schanz'schen Schraube 60 sind zwei im rechten Winkel zueinander stehende Flächen 61,62 geformt, die eine entsprechende Einkerbung oder Ausnehmung bilden. Die übrigbleibende Endfläche 63 erstreckt sich annähernd parallel zur Fläche 62. Ein Schaft 64 eines im übrigen nicht gezeigten Werkzeugs hat den gleichen Durchmesser wie die Schanz'sche Schraube 60. Er ist am freien Ende komplementär zur Schanz'schen Schraube 60 geformt. Da die Fläche 61 schräg zur Achse der Schraube 60 bzw. des Schaftes 64 verläuft, kann mit Hilfe des Schaftes 64 ein Zug auf die Schraube 60 aufgebracht werden. Der Eingriff der Teile ermöglicht auch die Übertragung eines Drehmoments. Damit die beschriebenen Teile miteinander in Eingriff gebracht werden, ist eine Gleithülse 65 vorgesehen, die gleitend auf dem Schaft 64 angeordnet ist und einen Längsschlitz 66 aufweist, in den ein Begrenzungsstift 67 hineinsteht, der im Schaft 64 radial befestigt ist. In den Figuren 11 und 12 ist die Verbindung von Schaft 64 und Schraube 60 dargestellt. Wird die Hülse 65 in Richtung des Pfeils 68 verschoben, kann die Schraube 60 befreit werden.

Die in den Figuren 11 und 12 beschriebene Ausbildung der Schanz'schen Schraube bzw. die gezeigte Vorrichtung ermöglichen die Handhabung relativ kurzer Schrauben mit Hilfe eines Werkzeugs. Normalerweise ist es nicht möglich, mit dem Werkzeug so nahe an die Wunde heranzugehen, wie es bei der Handhabung sehr kurzer Schanz'scher Schrauben notwendig wäre.

**Ansprüche**

1. Stützvorrichtung für die Wirbel der menschlichen Wirbelsäule, mit Haltekörpern, von denen Schanz'sche Schrauben klemmend aufgenommen werden, einem Verbindungselement für die Haltekörper, auf denen die Haltekörper in ihrer Relativlage verstellbar gelagert, jedoch in einer vorgegebenen Relativlage feststellbar sind, dadurch gekennzeichnet, daß die Haltekörper (22, 40) eine Gewindehülse (16, 41) aufweisen, die auf einem Ende eines Gewindebolzens (17) aufschraubbar ist, der Gewindebolzen (17) an den Enden gegenläufige Gewinde (19) aufweist, im Haltekörper (14, 40) eine Haltebuchse (22, 44) drehbar, jedoch axial unverstellbar gelagert ist, die gegebenenfalls Kraftangriffsflächen (23) für ein Werkzeug aufweist, die Haltebuchse (22, 44) eine eine Schanz'sche Schraube (60) aufnehmende Querdurchbohrung (20, 47) aufweist, die mit einer Bohrung (26, 43) im Haltekörper (14, 40) ausrichtbar ist, und im Haltekörper (14, 40) eine mit der Haltebuchse (22, 44) zusammenwirkende Feststellschraube (28, 53) angeordnet ist, die die Haltebuchse in einer eingenommenen Drehlage und zugleich die Schanz'sche Schraube (60) klemmend festlegt.

2. Vorrichtung nach Anspruch 1, dadurch gekennzeichnet, daß die Haltebuchse (22, 44) vorzugsweise diametral geschlitzt ist.

3. Vorrichtung nach Anspruch 1 oder 2, dadurch gekennzeichnet, daß sich die Durchbohrung (26) im Haltekörper (14) von der Mitte ausgehend nach außen jeweils in Achsrichtung erweitert.

4. Vorrichtung nach einem der Ansprüche 1 bis 3, dadurch gekennzeichnet, daß der Gewindebolzen (17) zwischen den Enden mit Kraftangriffsflächen (18) für ein Werkzeug versehen ist, vorzugsweise mit einem Sechskant.

5. Vorrichtung nach einem der Ansprüche 1 bis 4, dadurch gekennzeichnet, daß auf dem Gewindebolzen (17) Kontermuttern (30, 31) angeordnet sind.

6. Vorrichtung nach einem der Ansprüche 1 bis 5, dadurch gekennzeichnet, daß die Haltebuchse (44) innerhalb des Haltekörpers (40) von einem Spannring (49) umgeben ist, der einen radial nach außen weisenden Gewindeansatz (50) aufweist, der mit einer außen liegenden Klemmschraube (53) zusammenwirkt, der Gewindeansatz (50), die Klemmschraube (53) und der Spannring durchbohrt sind, wobei die Bohrungen koaxial zum Gewindeansatz sind, der Haltekörper (40) im Bereich der Durchbohrung für die Schanz'sche Schraube eine konvex gewölbte Klemmfläche (56) aufweist und zwischen Klemmfläche (56) und Klemmschraube (53) ein Klemmring (55) angeordnet ist, dessen der Klemmfläche (56) zugewandte Stirnfläche (55) entsprechend konkav gewölbt ist.

7. Vorrichtung nach Anspruch 6, dadurch gekennzeichnet, daß die Klemmfläche (56) und die konkave Stirnfläche (55) gezahnt sind.

8. Vorrichtung nach einem der Ansprüche 1 bis 7, dadurch gekennzeichnet, daß die Schanz'sche Schraube am freien Ende eine seitliche Ausnehmung (61, 62) aufweist derart, daß ein Werkzeugschaft (64) mit dazu korrespondierendem Ende sowohl ein Drehmoment als auch eine Zugkraft übertragen kann und auf den Werkzeugschaft (64) eine Hülse (65) begrenzt gleitend verschiebbar ist und in der einen Endstellung die verbundenen Endbereiche von Schanz'scher Schraube (60) und Werkzeugschaft (64) überbrückt und in der anderen Endstellung die Verbindung freigibt.

EP 0 303 773 A2

FIG.1

FIG.2

FIG.3

FIG.4

FIG.5

FIG.6

FIG.7

FIG.8

FIG.9

FIG.10

EP 0 303 773 A2

## FIG.11

## FIG.12